(19) Europäisches Patentamt
European Patent Office
Office européen des brevets

(11) EP 3 398 590 A1

(12) # EUROPÄISCHE PATENTANMELDUNG

(43) Veröffentlichungstag:
**07.11.2018 Patentblatt 2018/45**

(51) Int Cl.:
***A61K 9/20*** *(2006.01)* ***A61K 31/135*** *(2006.01)*
***A61K 31/4402*** *(2006.01)*

(21) Anmeldenummer: **17000750.4**

(22) Anmeldetag: **02.05.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **STADA ARZNEIMITTEL AG**
**61118 Bad Vilbel (DE)**

(72) Erfinder: **GREBE, Sarah-Lena**
**60385 Frankfurt am Main (DE)**

(74) Vertreter: **Kernebeck, Thomas**
**Kernebeck Patentanwalts GmbH**
**Stiftstraße 2**
**60313 Frankfurt am Main (DE)**

(54) **SCHMELZTABLETTE, ENTHALTEND EIN H1-ANTIHISTAMINIKA**

(57) Die vorliegende Erfindung betrifft eine Schmelztablette, insbesondere zur Behandlung von Schlafstörungen, umfassend als pharmazeutischen Wirkstoff ein $H_1$-Antihistaminika ausgewählt aus der Gruppe bestehend aus Doxylamin und dessen pharmazeutisch annehmbare Salze sowie Diphenhydramin und dessen pharmazeutisch annehmbare Salze. Die vorliegende Erfindung betrifft ferner die erfindungsgemäße Schmelztablette zur Verwendung zur Behandlung von Schlafstörungen sowie ein Verfahren zur Herstellung der erfindungsgemäßen Schmelztablette.

EP 3 398 590 A1

## Beschreibung

Die vorliegende Erfindung betrifft eine Schmelztablette, insbesondere zur Behandlung von Schlafstörungen, umfassend als pharmazeutischen Wirkstoff ein $H_1$-Antihistaminika ausgewählt aus der Gruppe bestehend aus Doxylamin und dessen pharmazeutisch annehmbare Salze sowie Diphenhydramin und dessen pharmazeutisch annehmbare Salze.

Doxylamin ((RS)-N,N-Dimethyl-2-[1-phenyl-1-(2-pyridyl)ethoxy]-ethylamin)

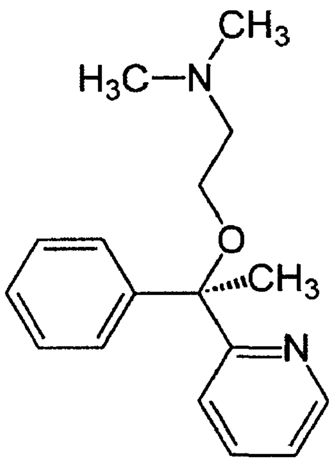

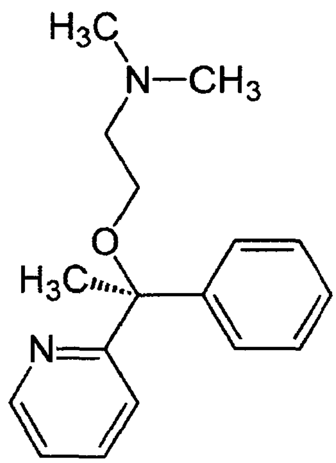

und Diphenhydramin (2-Benzhydryloxy-N,N-dimethyl-ethylamin)

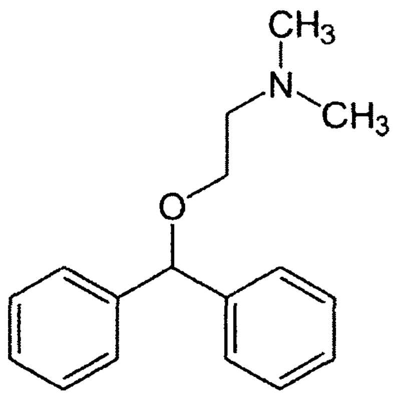

sind $H_1$-Antihistaminika, also Arzneistoffe, die durch eine Blockade von $H_1$-Rezeptoren die Wirkung von Histamin hemmen und somit antiallergisch wirken. Doxylamin und Diphenhydramin zählen zu den $H_1$-Antihistaminika der ersten Generation und werden gut in das zentrale Nervensystem aufgenommen. Aufgrund der Blockade zentraler $H_1$-Rezeptoren haben diese $H_1$-Antihistaminika zusätzlich einen sedierenden und antiemetischen Effekt und werden daher hauptsächlich in diesen Indikationsgebieten, nämlich als Sedativum (Schlaf- und Beruhigungsmittel) oder Antiemetikum (Mittel gegen Übelkeit und Erbrechen) eingesetzt.

Doxylamin-haltige Arzneimittel werden z.B. zur Ruhigstellung nervöser Patienten, zur Behandlung von Unruhe und Erregungszuständen bei Kindern und zur medikamentös erforderlichen Behandlung von Ein- und Durchschlafstörungen verwendet. Darüber hinaus werden Doxylamin-haltige Arzneimittel als Antiallergikum zur Behandlung allergischer Reaktionen der oberen und unteren Atemwege einschließlich Heuschnupfen und allergischer Bronchialkrämpfe sowie allergische Hauterkrankungen und Juckreiz eingesetzt. Weitere Einsatzgebiete in Kombination mit Vitamin B6 sind die Behandlung von Übelkeit und Erbrechen und in Kombination mit Paracetamol, Dextromethorphan und optional Ephedrin die symptomatische Behandlung von Erkältungskrankheiten.

Auch Diphenhydramin wird zur Behandlung von Ein- und Durchschlafstörungen eingesetzt. Darüber hinaus wird der in Rede stehende Wirkstoff zur Vorbeugung und symptomatischen Behandlung von Übelkeit und Erbrechen unterschiedlicher Ursache angewendet, insbesondere bei Reisekrankheit. Weiter findet Diphenhydramin Verwendung in der Behandlung von allergischen Symptomen und bei der Linderung von Juckreiz. Ferner hat Diphenhydramin die FDA-Zulassung zur symptomatischen Behandlung von Parkinson und extrapyramidalen Dyskinesien.

Ein Doxylamin-Präparat zur Behandlung von Schlafstörung wird beispielsweise unter dem Handelsnamen

Hoggar® Night im Markt angeboten. Dabei handelt es sich um Tabletten, die unzerkaut mit ausreichend Flüssigkeit durch Schlucken einzunehmen sind. Ein Diphenhydramin-Präperat zur Behandlung von Schlafstörungen wird unter dem Handelsnamen Hemodorm im Markt angeboten. Dabei handelt es sich ebenfalls um Tabletten, die unzerkaut mit Flüssigkeit durch Schlucken einzunehmen sind.

Hiervon ausgehend besteht die Aufgabe, eine Darreichungsform enthaltend ein $H_1$-Antihistaminika ausgewählt aus der Gruppe bestehend aus Doxylamin und Diphenhydramin bereitzustellen, die verhältnismäßig einfach eingenommen werden kann und somit eine höhere Patientenakzeptanz besitzt.

Diese Aufgabe wird erfindungsgemäß gelöst durch eine Schmelztablette, insbesondere zur Behandlung von Schlafstörungen, umfassend als pharmazeutischen Wirkstoff ein $H_1$-Antihistaminika ausgewählt aus der Gruppe bestehend aus Doxylamin und dessen pharmazeutisch annehmbare Salze sowie Diphenhydramin und dessen pharmazeutisch annehmbare Salze.

Die erfindungsgemäßen Schmelztabletten sind Tabletten, die im Mund verhältnismäßig schnell zergehen. Sie haben den Vorteil, dass sie auch von Personen mit Schluckbeschwerden sowie im Liegen unproblematisch eingenommen werden können und dass der Wirkstoff unter Umständen auch über die Mundschleimhaut resorbiert werden kann, weshalb bei den erfindungsgemäßen Schmelztabletten die pharmazeutische Wirkung vergleichsweise rasch eintreten kann. Darüber hinaus haben die erfindungsgemäßen Schmelztabletten den Vorteil, dass für ihre Einnahme ein Nachtrinken von Wasser in der Regel nicht erforderlich ist, so dass die Schmelztabletten auch für die Einnahme unterwegs besonders gut geeignet sind.

Wie bereits vorstehend ausgeführt, ist die erfindungsgemäße Tablette eine Schmelztablette, wofür auch der Begriff "orodispersible Tablette" im Stand der Technik verwendet wird. Das Europäische Arzneibuch (Ph.Eur.) definiert Schmelztabletten in der 8. Ausgabe, Grundwerk 2014, Band 1, auf Seite 1206 als nicht überzogene Tabletten, die im Mund behalten werden, wo sie sich schnell verteilen, bevor sie geschluckt werden, wobei Schmelztabletten eine Zerfallszeit gemäß Ph.Eur., 8. Ausgabe, Grundwerk 2014, Band 1, Seite 397 ff., Punkt 2.9.1 "Zerfallszeit von Tabletten und Kapseln" von kleiner/gleich 3 min aufweisen müssen. Die erfindungsgemäße Schmelztablette genügt dieser Definition.

In der erfindungsgemäßen Schmelztablette ist als Wirkstoff ein $H_1$-Antihistaminika ausgewählt aus der Gruppe bestehend aus Doxylamin und dessen pharmazeutisch annehmbare Salze sowie Diphenhydramin und dessen pharmazeutisch annehmbare Salze enthalten. Dabei liegt der Wirkstoff in der Schmelztablette in nicht-Ionenaustauscher gebundener Form, vorzugsweise in freier ungebundener Form, vor. Dadurch wird eine relativ kostengünstige Bereitstellung der erfindungsgemäßen Schmelztablette ermöglicht.

Dass der Wirkstoff in der erfindungsgemäßen Schmelztablette vorzugsweise in freier ungebundener Form vorliegt bedeutet dabei insbesondere, dass der Wirkstoff in der Schmelztablette als solches vorliegt und nicht etwa in chemisch gebundener Form wie etwa komplex (koordinativ) gebundener Form, beispielsweise komplex gebundener Form an einem Ionenaustauscher-Material wie einem Ionenaustauscherharz.

Entsprechend einer bevorzugten Ausführungsform der erfindungsgemäßen Schmelztablette ist es vorgesehen, dass die Schmelztablette eine Bruchfestigkeit F in einem Bereich von 30 N bis 120 N aufweist, bevorzugt eine Bruchfestigkeit F in einem Bereich von 40 N bis 110 N, mehr bevorzugt eine Bruchfestigkeit F in einem Bereich von 50 N bis 100 N, noch mehr bevorzugt eine Bruchfestigkeit F in einem Bereich von 60 N bis 100 N und weiter bevorzugt eine Bruchfestigkeit F in einem Bereich von 70 N oder 80 N bis 100 N. Dadurch wird gewährleistet, dass die erfindungsgemäße Schmelztablette mechanisch relativ stabil ist und daher weniger, wie ansonsten bei Schmelztabletten üblich, zu Beschädigungen neigt. Die erfindungsgemäße Schmelztablette kann daher beispielsweise in Durchdrück-Blisterpackungen angeboten werden und muss nicht, wie ansonsten üblich, in unpraktisch zu handhabenden Blistern mit einer Peelfolie verpackt werden, was die Patientenakzeptanz der erfindungsgemäßen Schmelztablette weiter erhöht.

Die Bruchfestigkeit F ist gemäß dem Europäischen Arzneibuch, 8. Ausgabe, Grundwerk 2014, Band 1, Seite 412, Punkt 2.9.8 "Bruchfestigkeit von Tabletten" zu bestimmen. Dabei ist es erfindungsgemäß bevorzugt, dass die Bruchfestigkeit F mittels eines Tablettenhärte-Testers durch uniaxiale vertikale Belastung zwischen zwei horizontalen Backen mit planparallelen Flächen gemessen wird durch Aufnahme einer kontrollierten Kraft-Weg-Kurve, wobei bei der Messung die Probe auf der unteren Backe platziert und worauf die obere Backe progressiv herabgefahren wird. Erfindungsgemäß besonders bevorzugt ist es, dass die Bruchfestigkeit F mittels eines Tabletten-Härtetesters mit der Bezeichnung Dr. Schleuniger Pharmatron Model 6 D bestimmt wird. Runde Tabletten, d.h. Tabletten mit einem kreisförmigen Querschnitt wie z.B. runde flache biplane, bikonvexe oder bikonkave Tabletten, werden bei der Bruchfestigkeitsmessung über den Steg (d.h. radial) zerbrochen.

Die Bruchfestigkeit F der erfindungsgemäßen Schmelztablette kann - wie dem Fachmann aus dem Stand der Technik bekannt - beispielsweise über Art und Menge der pharmazeutischen Hilfsstoffe wie etwa des eingesetzten Bindemittels sowie über die Größe der angewendeten Presskraft bei der Tablettierung eingestellt werden.

Die Bruchfestigkeit F einer Schmelztablette kann von ihrer Form und ihren Abmessungen abhängen. Es kann daher erfindungsgemäß bevorzugt sein, die mechanische Festigkeit der erfindungsgemäßen Schmelztablette als Druckfestigkeit $\sigma$ anzugeben, da die Druckfestigkeit $\sigma$ die Geometrie der Schmelztablette berücksichtigt und somit eine davon im Prinzip unabhängige Größe darstellt. Die Druckfestigkeit $\sigma$ wird mittels der experimentell bestimmten Bruchfestigkeit

F berechnet. Für zylinderförmige Schmelztabletten beispielsweise wird die Druckfestigkeit $\sigma$ mittels der nachstehenden mathematischen Beziehung berechnet (siehe "Die Tablette", A. Ritschel et al., 2. Auflage, Editio-Cantor-Verlag, 2002, ISBN 3-87193-228-0):

$$\sigma = \frac{2\,F}{\pi\,D\,t}$$

in welcher

- $\sigma$ die Druckfestigkeit ist;
- F die Bruchfestigkeit ist;
- $\pi$ die Zahl pi ist;
- D der Tablettendurchmesser ist;
- t die Tablettendicke ist.

Für gewölbte Schmelztabletten beispielsweise wird die Druckfestigkeit $\sigma$ mittels der nachstehenden mathematischen Beziehung berechnet (siehe "Die Tablette", A. Ritschel et al., 2. Auflage, Editio-Cantor-Verlag, 2002, ISBN 3-87193-228-0):

$$\sigma = \frac{10F}{\pi D^2}\left(2{,}84\frac{t}{D} - 0{,}126\frac{t}{W} + 3{,}15\,\frac{W}{D} + 0{,}01\right)^{-1}$$

in welcher

- $\sigma$ die Druckfestigkeit ist;
- F die Bruchfestigkeit ist;
- $\pi$ die Zahl pi ist;
- D der Tablettendurchmesser ist;
- t die Dicke über alles;
- W die Dicke des zentralen Zylinders ist.

Gemäß einer weiter bevorzugten Ausführungsform der erfindungsgemäßen Schmelztablette ist es vorgesehen, dass die Schmelztablette eine Druckfestigkeit $\sigma$ von größer/gleich 0,4 $N/mm^2$ aufweist, bevorzugt eine Druckfestigkeit $\sigma$ in einem Bereich von 0,45 $N/mm^2$ bis 2,5 $N/mm^2$, weiter bevorzugt eine Druckfestigkeit $\sigma$ in einem Bereich von 0,5 $N/mm^2$ bis 1,50 $N/mm^2$ und besonders bevorzugt eine Druckfestigkeit $\sigma$ in einem Bereich von 0,70 $N/mm^2$ bis 1,30 $N/mm^2$.

Gemäß einer weiter bevorzugten Ausführungsform der erfindungsgemäßen Schmelztablette ist es vorgesehen, dass die Schmelztablette eine Zerfallszeit in einem Bereich von 20 s oder 60 s bis 180 s aufweist, bevorzugt eine Zerfallszeit in einem Bereich von 70 s bis 150 s und besonders bevorzugt eine Zerfallszeit in einem Bereich von 80 s bis 120 s. Die Zerfallszeit ist gemäß Ph.Eur., 8. Ausgabe, Grundwerk 2014, Band 1, Seite 397 ff., Punkt 2.9.1 "Zerfallszeit von Tabletten und Kapseln" zu bestimmen, wobei als Medium vorzugsweise Wasser eingesetzt wird.

Entsprechend einer weiter bevorzugten Ausführungsform der erfindungsgemäßen Schmelztablette ist es vorgesehen, dass die Schmelztablette eine Friabilität von kleiner/gleich 1 % aufweist, bevorzugt eine Friabilität von kleiner/gleich 0,5 % und besonders bevorzugt eine Friabilität von 0,1 % bis 0,4 %. Die Friabilität ist gemäß Ph.Eur, 8. Ausgabe, Grundwerk 2014, Band 1, Seite 411 ff., Punkt 2.9.7 "Friabilität von nicht überzogenen Tabletten" zu bestimmen.

Nach einer weiter bevorzugten Ausführungsform der erfindungsgemäßen Schmelztablette ist es vorgesehen, dass die Schmelztablette direkttablettiert ist. Dadurch wird eine verhältnismäßig kostengünstige Bereitstellung der erfindungsgemäßen Schmelztablette gewährleistet.

Gemäß einer weiter bevorzugten Ausführungsform der erfindungsgemäßen Schmelztablette ist es vorgesehen, dass die Schmelztablette frei von einem Ionenaustauscher-Material ist, insbesondere frei von einem Ionenaustauscherharz. Zur weitgehenden Unterdrückung des bitteren Geschmacks des wirkstoffs in der erfindungsgemäßen Zusammensetzung ist der Einsatz eines relativ teuren Ionenaustauscher-Materials, an welchem der Wirkstoff komplex gebunden ist, nicht notwendig.

Gemäß einer weiter bevorzugten Ausführungsform der erfindungsgemäßen Schmelztablette ist es vorgesehen, dass die Schmelztablette frei von Pyridoxin und frei von einem Salz des Pyridoxins ist.

Nach einer weiter bevorzugten Ausführungsform der erfindungsgemäßen Schmelztablette ist es vorgesehen, dass der Wirkstoff der einzige pharmazeutische Wirkstoff in der Schmelztablette ist. Dadurch wird die Verwendung der

erfindungsgemäßen Zusammensetzung zur Behandlung von Schlafstörungen gewährleistet.

Entsprechend einer weiter bevorzugten Ausführungsform der erfindungsgemäßen Schmelztablette ist es vorgesehen, dass der Wirkstoff Doxylaminsuccinat ist, insbesondere Doxylaminsuccinat-Hemihydrat. Doxylaminsuccinat weist in der erfindungsgemäßen Schmelztablette eine verhältnismäßig hohe chemische Stabilität auf, weshalb die Schmelztablette durch eine relative lange Haltbarkeit gekennzeichnet ist.

Nach einer weiter bevorzugten Ausführungsform der erfindungsgemäßen Schmelztablette ist es vorgesehen, dass der Wirkstoff Diphenhydraminhydrochlorid ist. Diphenhydraminhydrochlorid weist in der erfindungsgemäßen Schmelztablette eine verhältnismäßig hohe chemische Stabilität auf, weshalb die Schmelztablette durch eine relative lange Haltbarkeit gekennzeichnet ist.

Gemäß einer weiter bevorzugten Ausführungsform der erfindungsgemäßen Schmelztablette ist es vorgesehen, dass der Wirkstoff in der Schmelztablette in kristalliner Form vorliegt. In kristalliner Form weist der Wirkstoff in der erfindungsgemäßen Schmelztablette eine verhältnismäßig hohe chemische Stabilität auf, weshalb die Schmelztablette durch eine relative lange Haltbarkeit gekennzeichnet ist.

Nach einer weiter bevorzugten Ausführungsform der erfindungsgemäßen Schmelztablette ist es vorgesehen, dass der Wirkstoff in der Schmelztablette in einer Menge in einem Bereich von 1 mg bis 500 mg enthalten ist, bevorzugt in einer Menge in einem Bereich von 5 mg bis 100 mg, mehr bevorzugt in einer Menge in einem Bereich von 10 mg bis 50 mg und weiter bevorzugt in einer Menge von 25 mg oder von 50 mg.

Gemäß einer weiter bevorzugten Ausführungsform der erfindungsgemäßen Schmelztablette ist es vorgesehen, dass die Schmelztablette ein Geschmacksmaskierungsmittel umfasst. Dadurch wird eine weitgehende Unterdrückung des bitteren Geschmacks des Wirkstoffs in der erfindungsgemäßen Zusammensetzung sichergestellt.

Entsprechend einer weiter bevorzugten Ausführungsform der erfindungsgemäßen Schmelztablette ist es vorgesehen, dass das Geschmacksmaskierungsmittel zumindest ein Cyclodextrin umfasst, bevorzugt zumindest ein Cyclodextrin und zumindest ein Süßungsmittel, weiter bevorzugt zumindest ein Cyclodextrin und zumindest ein Aromastoff und besonders bevorzugt zumindest ein Cyclodextrin, zumindest ein Süßungsmittel sowie zumindest einen Aromastoff. Es konnte festgestellt werden, dass die genannten Alternativen von Geschmacksmaskierungsmitteln besonders geeignet zur weitgehenden Unterdrückung des bitteren Geschmacks des Wirkstoffs in der erfindungsgemäßen Schmelztablette sind. In diesem Zusammenhang ist es insbesondere bevorzugt, wenn das Geschmacksmaskierungsmittel eine Kombination von β-Cyclodextrin, Sucralose und einem Aromastoff umfasst, besonders bevorzugt eine Kombination von β-Cyclodextrin, Sucralose, Erdbeer-Aroma und Rhababer-Vanille-Aroma.

Gemäß einer weiter bevorzugten Ausführungsform der erfindungsgemäßen Schmelztablette ist es vorgesehen, dass die Schmelztablette einen wasserlöslichen und einen wasserunlöslichen pharmazeutischen Füllstoff umfasst. Füllstoffe dienen dem Zweck, das Volumen von Tabletten auf eine geeignete Größe zu erhöhen, speziell bei niederdosierten Tabletten. Die Füllstoffmenge hängt von seiner Art, der Größe der Tablette und der Menge des Wirkstoffs ab. Unter wasserlöslich wird eine Wasserlöslichkeit bei einer Temperatur von 15 °C bis 25 °C von mehr als 33 mg pro ml verstanden, bevorzugt eine Wasserlöslichkeit von 100 mg pro ml bis 1000 mg pro ml. Unter wasserunlöslich wird eine Wasserlöslichkeit bei einer Temperatur von 15 °C bis 25 °C von weniger als 1 mg pro ml verstanden, bevorzugt eine Wasserlöslichkeit von weniger als 0,1 mg pro ml.

Entsprechend einer weiter bevorzugten Ausführungsform der erfindungsgemäßen Schmelztablette ist es vorgesehen, dass der wasserlösliche Füllstoff ausgewählt ist aus der Gruppe bestehend aus einem Zucker und einem Zuckeralkohol.

Gemäß einer weiter bevorzugten Ausführungsform der erfindungsgemäßen Schmelztablette ist es vorgesehen, dass der Zucker bzw. der Zuckeralkohol ausgewählt ist aus der Gruppe bestehend aus Dextrose, Lactose, Mannitol, Sorbitol, Saccharose und Xylitol.

Entsprechend einer weiter bevorzugten Ausführungsform der erfindungsgemäßen Schmelztablette ist es vorgesehen, dass der wasserlösliche Füllstoff Mannitol ist.

Gemäß einer weiter bevorzugten Ausführungsform der erfindungsgemäßen Schmelztablette ist es vorgesehen, dass der wasserunlösliche Füllstoff ausgewählt ist aus der Gruppe bestehend aus Aluminiumhydroxid, Bariumsulfat, Calciumcarbonat, Calciumphosphat, Calciumsulfat, Magnesiumcarbonat, Magnesiumtrisilicat, Kaolin, Cellulose, vorzugsweise mikrokristalline Cellulose, und Stärke.

Entsprechend einer weiter bevorzugten Ausführungsform der erfindungsgemäßen Schmelztablette ist es vorgesehen, dass der wasserunlösliche Füllstoff Stärke ist.

Nach einer weiter bevorzugten Ausführungsform der erfindungsgemäßen Schmelztablette ist es vorgesehen, dass der wasserlösliche und der wasserunlösliche pharmazeutische Füllstoff miteinander vermischt in Form eines Granulats vorliegen.

Entsprechend einer weiter bevorzugten Ausführungsform der erfindungsgemäßen Schmelztablette ist es vorgesehen, dass die Schmelztablette ein zur Direkttablettierung geeignetes Bindemittel umfasst, vorzugsweise mikrokristalline Cellulose.

Gemäß einer weiter bevorzugten Ausführungsform der erfindungsgemäßen Schmelztablette ist es vorgesehen,

dass die Schmelztablette einen oder mehrere pharmazeutische Hilfsstoffe umfasst, ausgewählt aus der Gruppe bestehend aus einem Sprengmittel, Schmiermittel und einem Fließregulierungsmittel.

Eine typische, durch die vorliegende Erfindung bereitgestellte Schmelztablette ist eine Schmelztablette zur Behandlung von Schlafstörungen, umfassend als einzigen pharmazeutischen Wirkstoff 10 mg bis 50 mg Doxylaminsuccinat in nicht-Ionenaustauscher gebundener Form, vorzugsweise in freier ungebundener Form, sowie ein Geschmacksmaskierungsmittel, insbesondere ein Geschmacksmaskierungsmittel umfassend zumindest ein Cyclodextrin, zumindest ein Süßungsmittel sowie zumindest einen Aromastoff, vorzugsweise ein Geschmacksmaskierungsmittel umfassend β-Cyclodextrin, Sucralose und ein Aromastoff, besonders bevorzugt ein Geschmacksmaskierungsmittel umfassend β-Cyclodextrin, Sucralose, Erdbeer-Aroma und Rhababer-Vanille-Aroma, wobei die Schmelztablette eine Bruchfestigkeit F in einem Bereich von 50 N bis 100 N aufweist.

Eine weitere typische, durch die vorliegende Erfindung bereitgestellte Schmelztablette ist eine Schmelztablette zur Behandlung von Schlafstörungen, umfassend als einzigen pharmazeutischen Wirkstoff 25 mg bis 75 mg Diphenhydraminhydrochlorid, vorzugsweise in nicht-Ionenaustauscher gebundener Form, bevorzugt in freier ungebundener Form, sowie ein Geschmacksmaskierungsmittel, insbesondere ein Geschmacksmaskierungsmittel umfassend zumindest ein Cyclodextrin, zumindest ein Süßungsmittel sowie zumindest einen Aromastoff, vorzugsweise ein Geschmacksmaskierungsmittel umfassend β-Cyclodextrin, Sucralose und ein Aromastoff, besonders bevorzugt ein Geschmacksmaskierungsmittel umfassend β-Cyclodextrin, Sucralose, Erdbeer-Aroma und Rhababer-Vanille-Aroma, wobei die Schmelztablette eine Bruchfestigkeit F in einem Bereich von 50 N bis 100 N aufweist.

Die vorliegende Erfindung betrifft ferner eine erfindungsgemäße Schmelztablette zur Verwendung zur Behandlung von Schlafstörungen.

Die vorliegende Erfindung betrifft darüber hinaus ein Verfahren zur Herstellung einer erfindungsgemäßen Schmelztablette, umfassend die Schritte:

- Bereitstellen des Wirkstoffs;
- Vermischen des Wirkstoffs mit einem pharmazeutischen Hilfsstoff, der zur Direkttablettierung geeignet ist, wobei ein Gemisch erhalten wird;
- Verpressen des Gemisches unter Erhalt einer Schmelztablette.

Die nachfolgenden Ausführungsbeispiele dienen der Erläuterung der Erfindung.

**Ausführungsbeispiel 1:**

Es wurden Schmelztabletten enthaltend als Wirkstoff 25 mg Doxylaminsuccinat hergestellt. Die Schmelztabletten weisen die in der Tabelle 1 angegebene Zusammensetzung auf:

**Tabelle 1:**

| **Substanz** | **Menge pro Tablette [mg]** | **Funktion der Substanz** |
|---|---|---|
| | | |
| Doxylaminsuccinat | 25,0 | Wirkstoff |
| Pearlitol® Flash (eine Mannitol/Maisstärke-Zusammensetzung) | 370,3 | Füllstoff |
| Natriumstearylfumarat | 6,0 | Schmiermittel |
| hochdisperses Siliciumdioxid | 6,0 | Fließregulierungsmittel |
| mikrokristalline Cellulose | 90,0 | Bindemittel |
| Crospovidon | 30,0 | Sprengmittel |
| Sucralose | 1,0 | Geschmacksmaskierungsmittel (Süßungsmittel) |
| Erdbeer-Aroma | 5,4 | Geschmacksmaskierungsmittel (Aromastoff) |
| Rhababer-Vanille-Aroma | 6,3 | Geschmacksmaskierungsmittel (Aromastoff) |
| β-Cyclodextrin | 60,0 | Geschmacksmaskierungsmittel (Cyclodextrin) |
| **Gesamtgewicht der Schmelztablette** | 600,0 | |

Die Schmelztabletten gemäß Tabelle 1 wurden wie folgt hergestellt: zunächst wurden der Wirkstoff Doxylaminsuccinat und das Geschmacksmaskierungsmittel β-Cyclodextrin jeweils gesiebt und dann miteinander vermischt unter Erhalt eines wirkstoff-Gemisches. Daraufhin wurden der Füllstoff in Form der Mannitol/Maisstärke-Zusammensetzung (Pearlitol® Flash), das Fließregulierungsmittel Siliciumdioxid, das Bindemittel mikrokristalline Cellulose, das Sprengmittel Crospovidon sowie die Geschmacksmaskierungsmittel Sucralose, Erdbeer-Aroma und Rhababer-Vanille-Aroma jeweils gesiebt und dann ebenfalls miteinander vermischt unter Erhalt eines Hilfsstoff-Gemisches.

Die wie vorstehend beschrieben hergestellten Wirkstoff- und Hilfsstoff-Gemische wurden unter Zusatz des Schmiermittels Natriumstearylfumarat miteinander zu einer finalen Tablettenmischung vermischt, die mittels einer konventionellen Tablettenpresse zu runden biplanen Schmelztabletten mit einem Durchmesser von 12,7 mm und einer Dicke von 4,5 mm direkttablettiert wurde.

Bruchfestigkeit F (Ph.Eur. 2.9.8): Mittelwert: 98,1 N (Extremwerte: (Min) 88 N und (Max) 106 N)
Druckfestigkeit $\sigma$: 1,09 $N/mm^2$
Zerfallszeit (Ph.Eur. 2.9.1): 104 s

Die Bruchfestigkeit F der Tabletten gemäß Ph.Eur. 2.9.8 wurde mittels eines Tabletten-Härtetesters mit der Bezeichnung Dr. Schleuniger Pharmatron Model 6 D vermessen. Die Ausrichtung der Tabletten war bei der Vermessung derart, dass sie über den Steg (d.h. radial) zerbrochen wurden.

Die Zerfallszeit gemäß Ph.Eur. 2.9.1 wurde mittels des Mediums Wasser bestimmt.

**Ausführungsbeispiel 2:**

Es wurden Schmelztabletten enthaltend als Wirkstoff 50 mg Diphenhydraminhydrochlorid hergestellt. Die Schmelztabletten weisen die in der Tabelle 2 angegebene Zusammensetzung auf:

**Tabelle 2:**

| **Substanz** | **Menge pro Tablette [mg]** | **Funktion der Substanz** |
|---|---|---|
| | | |
| Diphenhydraminhydrochlorid | 50,0 | Wirkstoff |
| Pearlitol® Flash (eine Mannitol/Maisstärke-Zusammensetzung) | 345,3 | Füllstoff |
| Natriumstearylfumarat | 6,0 | Schmiermittel |
| hochdisperses Siliciumdioxid | 6,0 | Fließregulierungsmittel |
| mikrokristalline Cellulose | 90,0 | Bindemittel |
| Crospovidon | 30,0 | Sprengmittel |
| Sucralose | 1,0 | Geschmacksmaskierungsmittel (Süßungsmittel) |
| Erdbeer-Aroma | 5,4 | Geschmacksmaskierungsmittel (Aromastoff) |
| Rhababer-Vanille-Aroma | 6,3 | Geschmacksmaskierungsmittel (Aromastoff) |
| β-Cyclodextrin | 60,0 | Geschmacksmaskierungsmittel (Cyclodextrin) |
| **Gesamtgewicht der Schmelztablette** | 600,0 | |

Die Schmelztabletten gemäß Tabelle 2 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

Bruchfestigkeit F (Ph.Eur. 2.9.8): Mittelwert: 97,6 N (Extremwerte: (Min) 87 N und (Max) 105 N)

(fortgesetzt)

| | |
|---|---|
| Druckfestigkeit σ: | 1,09 $N/mm^2$ |
| Zerfallszeit (Ph.Eur. 2.9.1): | 106 s |

**Ausführungsbeispiel 3:**

Es wurden Schmelztabletten enthaltend als Wirkstoff 25 mg Doxylaminsuccinat hergestellt. Die Schmelztabletten weisen die in der Tabelle 3 angegebene Zusammensetzung auf:

**Tabelle 3:**

| **Substanz** | **Menge pro Tablette [mg]** | **Funktion der Substanz** |
|---|---|---|
| | | |
| Doxylaminsuccinat | 25,0 | Wirkstoff |
| Pearlitol® Flash (eine Mannitol/Maisstärke-Zusammensetzung) | 345,1 | Füllstoff |
| Natriumstearylfumarat | 9,0 | Schmiermittel |
| hochdisperses Siliciumdioxid | 4,5 | Fließregulierungsmittel |
| mikrokristalline Cellulose | 80,0 | Bindemittel |
| Crospovidon | 25,0 | Sprengmittel |
| Sucralose | 1,5 | Geschmacksmaskierungsmittel (Süßungsmittel) |
| Erdbeer-Aroma | 4,2 | Geschmacksmaskierungsmittel (Aromastoff) |
| Rhababer-Vanille-Aroma | 5,7 | Geschmacksmaskierungsmittel (Aromastoff) |
| β-Cyclodextrin | 100,0 | Geschmacksmaskierungsmittel (Cyclodextrin) |
| **Gesamtgewicht der Schmelztablette** | 600,0 | |

Die Schmelztabletten gemäß Tabelle 3 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 89,7 N<br>(Extremwerte: (Min) 83 N und (Max) 101 N) |
| Druckfestigkeit σ: | 0,99 $N/mm^2$ |
| Zerfallszeit (Ph.Eur. 2.9.1): | 101 s |

**Ausführungsbeispiel 4:**

Es wurden Schmelztabletten enthaltend als Wirkstoff 50 mg Diphenhydraminhydrochlorid hergestellt. Die Schmelztabletten weisen die in der Tabelle 4 angegebene Zusammensetzung auf:

**Tabelle 4:**

| **Substanz** | **Menge pro Tablette [mg]** | **Funktion der Substanz** |
|---|---|---|
| | | |
| Diphenhydraminhydrochlorid | 50,0 | Wirkstoff |
| Pearlitol® Flash (eine Mannitol/Maisstärke-Zusammensetzung) | 320,1 | Füllstoff |

(fortgesetzt)

| Substanz | Menge pro Tablette [mg] | Funktion der Substanz |
|---|---|---|
| Natriumstearylfumarat | 9,0 | Schmiermittel |
| hochdisperses Siliciumdioxid | 4,5 | Fließregulierungsmittel |
| mikrokristalline Cellulose | 80,0 | Bindemittel |
| Crospovidon | 25,0 | Sprengmittel |
| Sucralose | 1,5 | Geschmacksmaskierungsmittel (Süßungsmittel) |
| Erdbeer-Aroma | 4,2 | Geschmacksmaskierungsmittel (Aromastoff) |
| Rhababer-Vanille-Aroma | 5,7 | Geschmacksmaskierungsmittel (Aromastoff) |
| β-Cyclodextrin | 100,0 | Geschmacksmaskierungsmittel (Cyclodextrin) |
| **Gesamtgewicht der Schmelztablette** | 600,0 | |

Die Schmelztabletten gemäß Tabelle 4 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 91,2 N<br>(Extremwerte: (Min) 84 N und (Max) 101 N) |
| Druckfestigkeit σ: | 1,02 $N/mm^2$ |
| Zerfallszeit (Ph.Eur. 2.9.1): | 102 s |

**Ausführungsbeispiel 5:**

Es wurden Schmelztabletten enthaltend als Wirkstoff 25 mg Doxylaminsuccinat hergestellt. Die Schmelztabletten weisen die in der Tabelle 5 angegebene Zusammensetzung auf:

**Tabelle 5:**

| Substanz | Menge pro Tablette [mg] | Funktion der Substanz |
|---|---|---|
| | | |
| Doxylaminsuccinat | 25,0 | Wirkstoff |
| Pearlitol® Flash (eine Mannitol/Maisstärke-Zusammensetzung) | 310,9 | Füllstoff |
| Natriumstearylfumarat | 6,0 | Schmiermittel |
| hochdisperses Siliciumdioxid | 6,0 | Fließregulierungsmittel |
| mikrokristalline Cellulose | 90,0 | Bindemittel |
| Crospovidon | 30,0 | Sprengmittel |
| Sucralose | 1,3 | Geschmacksmaskierungsmittel (Süßungsmittel) |
| Erdbeer-Aroma | 4,8 | Geschmacksmaskierungsmittel (Aromastoff) |
| Rhababer-Vanille-Aroma | 6,0 | Geschmacksmaskierungsmittel (Aromastoff) |

(fortgesetzt)

| Substanz | Menge pro Tablette [mg] | Funktion der Substanz |
|---|---|---|
| β-Cyclodextrin | 120,0 | Geschmacksmaskierungsmittel (Cyclodextrin) |
| **Gesamtgewicht der Schmelztablette** | 600,0 | |

Die Schmelztabletten gemäß Tabelle 5 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 95,3 N<br>(Extremwerte: (Min) 86 N und (Max) 104 N) |
| Druckfestigkeit σ: | 1,06 N/mm$^2$ |
| Zerfallszeit (Ph.Eur. 2.9.1): | 102 s |

## Ausführungsbeispiel 6:

Es wurden Schmelztabletten enthaltend als Wirkstoff 50 mg Diphenhydraminhydrochlorid hergestellt. Die Schmelztabletten weisen die in der Tabelle 6 angegebene Zusammensetzung auf:

**Tabelle 6:**

| Substanz | Menge pro Tablette [mg] | Funktion der Substanz |
|---|---|---|
| | | |
| Diphenhydraminhydrochlorid | 50,0 | Wirkstoff |
| Pearlitol® Flash (eine Mannitol/Maisstärke-Zusammensetzung) | 285,9 | Füllstoff |
| Natriumstearylfumarat | 6,0 | Schmiermittel |
| hochdisperses Siliciumdioxid | 6,0 | Fließregulierungsmittel |
| mikrokristalline Cellulose | 90,0 | Bindemittel |
| Crospovidon | 30,0 | Sprengmittel |
| Sucralose | 1,3 | Geschmacksmaskierungsmittel (Süßungsmittel) |
| Erdbeer-Aroma | 4,8 | Geschmacksmaskierungsmittel (Aromastoff) |
| Rhababer-Vanille-Aroma | 6,0 | Geschmacksmaskierungsmittel (Aromastoff) |
| β-Cyclodextrin | 120,0 | Geschmacksmaskierungsmittel (Cyclodextrin) |
| **Gesamtgewicht der Schmelztablette** | 600,0 | |

Die Schmelztabletten gemäß Tabelle 6 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 94,8 N<br>(Extremwerte: (Min) 85 |

(fortgesetzt)

| | |
|---|---|
| | N und (Max) 102 N) |
| Druckfestigkeit σ: | 1,06 N/mm$^2$ |
| Zerfallszeit (Ph.Eur. 2.9.1): | 98 s |

**Ausführungsbeispiel 7:**

Es wurden Schmelztabletten enthaltend als Wirkstoff 25 mg Doxylaminsuccinat hergestellt. Die Schmelztabletten weisen die in der Tabelle 7 angegebene Zusammensetzung auf:

**Tabelle 7:**

| **Substanz** | **Menge pro Tablette [mg]** | **Funktion der Substanz** |
|---|---|---|
| | | |
| Doxylaminsuccinat | 25,0 | Wirkstoff |
| Pearlitol® Flash (eine Mannitol/Maisstärke-Zusammensetzung) | 363,8 | Füllstoff |
| Natriumstearylfumarat | 6,0 | Schmiermittel |
| hochdisperses Siliciumdioxid | 3,0 | Fließregulierungsmittel |
| mikrokristalline Cellulose | 60,0 | Bindemittel |
| Crospovidon | 30,0 | Sprengmittel |
| Sucralose | 0,9 | Geschmacksmaskierungsmittel (Süßungsmittel) |
| Erdbeer-Aroma | 4,1 | Geschmacksmaskierungsmittel (Aromastoff) |
| Rhababer-Vanille-Aroma | 7,2 | Geschmacksmaskierungsmittel (Aromastoff) |
| β-Cyclodextrin | 100,0 | Geschmacksmaskierungsmittel (Cyclodextrin) |
| **Gesamtgewicht der Schmelztablette** | 600,0 | |

Die Schmelztabletten gemäß Tabelle 7 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 89,4 N (Extremwerte: (Min) 82 N und (Max) 98 N) |
| Druckfestigkeit σ: | 1,00 N/mm$^2$ |
| Zerfallszeit (Ph.Eur. 2.9.1): | 100 s |

**Ausführungsbeispiel 8:**

Es wurden Schmelztabletten enthaltend als Wirkstoff 25 mg Doxylaminsuccinat hergestellt. Die Schmelztabletten weisen die in der Tabelle 8 angegebene Zusammensetzung auf:

**Tabelle 8:**

| **Substanz** | **Menge pro Tablette [mg]** | **Funktion der Substanz** |
|---|---|---|
| | | |

(fortgesetzt)

| Substanz | Menge pro Tablette [mg] | Funktion der Substanz |
|---|---|---|
| Doxylaminsuccinat | 25,0 | Wirkstoff |
| Pearlitol® Flash (eine Mannitol/Maisstärke-Zusammensetzung) | 344,5 | Füllstoff |
| Natriumstearylfumarat | 6,0 | Schmiermittel |
| hochdisperses Siliciumdioxid | 6,0 | Fließregulierungsmittel |
| mikrokristalline Cellulose | 90,0 | Bindemittel |
| Crospovidon | 35,0 | Sprengmittel |
| Sucralose | 1,0 | Geschmacksmaskierungsmittel (Süßungsmittel) |
| Erdbeer-Aroma | 5,8 | Geschmacksmaskierungsmittel (Aromastoff) |
| Rhababer-Vanille-Aroma | 6,7 | Geschmacksmaskierungsmittel (Aromastoff) |
| β-Cyclodextrin | 80,0 | Geschmacksmaskierungsmittel (Cyclodextrin) |
| **Gesamtgewicht der Schmelztablette** | 600,0 | |

Die Schmelztabletten gemäß Tabelle 8 wurden analog Ausführungsbeispiel 1 hergestellt - unter Erhalt von runden biplanen Schmelztabletten mit einem Durchmesser von 12,7 mm und einer Dicke von 4,5 mm - und analog Ausführungsbeispiel 1 vermessen.

| | |
|---|---|
| Bruchfestigkeit F (Ph.Eur. 2.9.8): | Mittelwert: 91,3 N (Extremwerte: (Min) 84 N und (Max) 102 N) |
| Druckfestigkeit σ: | 1,02 N/mm$^2$ |
| Zerfallszeit (Ph.Eur. 2.9.1): | 93 s |

## Patentansprüche

1. Schmelztablette, insbesondere zur Behandlung von Schlafstörungen, umfassend als pharmazeutischen Wirkstoff ein $H_1$-Antihistaminika ausgewählt aus der Gruppe bestehend aus Doxylamin und dessen pharmazeutisch annehmbare Salze sowie Diphenhydramin und dessen pharmazeutisch annehmbare Salze.

2. Schmelztablette nach Anspruch 1, **dadurch gekennzeichnet, dass** die Schmelztablette eine Bruchfestigkeit F in einem Bereich von 30 N bis 120 N aufweist, bevorzugt eine Bruchfestigkeit F in einem Bereich von 40 N bis 110 N, mehr bevorzugt eine Bruchfestigkeit F in einem Bereich von 50 N bis 100 N, noch mehr bevorzugt eine Bruchfestigkeit F in einem Bereich von 60 N bis 100 N und weiter bevorzugt eine Bruchfestigkeit F in einem Bereich von 70 N oder 80 N bis 100 N.

3. Schmelztablette nach einem der voranstehenden Ansprüche, **dadurch gekennzeichnet, dass** die Schmelztablette eine Druckfestigkeit σ von größer/gleich 0,4 N/mm$^2$ aufweist, bevorzugt eine Druckfestigkeit σ in einem Bereich von 0,45 N/mm$^2$ bis 2,5 N/mm$^2$, weiter bevorzugt eine Druckfestigkeit σ in einem Bereich von 0,5 N/mm$^2$ bis 1,50 N/mm$^2$ und besonders bevorzugt eine Druckfestigkeit σ in einem Bereich von 0,70 N/mm$^2$ bis 1,30 N/mm$^2$.

4. Schmelztablette nach einem der voranstehenden Ansprüche, **dadurch gekennzeichnet, dass** die Schmelztablette eine Zerfallszeit in einem Bereich von 20 s oder 60 s bis 180 s aufweist, bevorzugt eine Zerfallszeit in einem Bereich von 70 s bis 150 s und besonders bevorzugt eine Zerfallszeit in einem Bereich von 80 s bis 120 s.

5. Schmelztablette nach einem der voranstehenden Ansprüche, **dadurch gekennzeichnet, dass** die Schmelztablette eine Friabilität von kleiner/gleich 1 % aufweist.

**6.** Schmelztablette nach einem der voranstehenden Ansprüche, **dadurch gekennzeichnet, dass** die Schmelztablette direkttablettiert ist.

**7.** Schmelztablette nach einem der voranstehenden Ansprüche, **dadurch gekennzeichnet, dass** die Schmelztablette frei von einem Ionenaustauscher-Material ist.

**8.** Schmelztablette nach einem der voranstehenden Ansprüche, **dadurch gekennzeichnet, dass** der Wirkstoff der einzige pharmazeutische Wirkstoff in der Schmelztablette ist.

**9.** Schmelztablette nach einem der voranstehenden Ansprüche, **dadurch gekennzeichnet, dass** der Wirkstoff Doxylaminsuccinat oder Diphenhydraminhydrochlorid ist.

**10.** Schmelztablette nach einem der voranstehenden Ansprüche, **dadurch gekennzeichnet, dass** der Wirkstoff in der Schmelztablette in kristalliner Form vorliegt.

**11.** Schmelztablette nach einem der voranstehenden Ansprüche, **dadurch gekennzeichnet, dass** der Wirkstoff in der Schmelztablette in einer Menge in einem Bereich von 1 mg bis 500 mg enthalten ist, bevorzugt in einer Menge in einem Bereich von 5 mg bis 100 mg, mehr bevorzugt in einer Menge in einem Bereich von 10 mg bis 50 mg und weiter bevorzugt in einer Menge von 25 mg oder 50 mg.

**12.** Schmelztablette nach einem der voranstehenden Ansprüche, **dadurch gekennzeichnet, dass** die Schmelztablette ein Geschmacksmaskierungsmittel umfasst.

**13.** Schmelztablette nach Anspruch 12, **dadurch gekennzeichnet, dass** das Geschmacksmaskierungsmittel zumindest ein Cyclodextrin, zumindest ein Süßungsmittel sowie zumindest einen Aromastoff umfasst.

**14.** Schmelztablette nach einem der Ansprüche 1 bis 13 zur Verwendung zur Behandlung von Schlafstörungen.

**15.** Verfahren zur Herstellung einer Schmelztablette nach einem der Ansprüche 1 bis 13, umfassend die Schritte:

- Bereitstellen des Wirkstoffs;
- Vermischen des Wirkstoffs mit einem pharmazeutischen Hilfsstoff, der zur Direkttablettierung geeignet ist, wobei ein Gemisch erhalten wird;
- Verpressen des Gemisches unter Erhalt einer Schmelztablette.

Europäisches Patentamt
European Patent Office
Office européen des brevets

# EUROPÄISCHER RECHERCHENBERICHT

Nummer der Anmeldung
EP 17 00 0750

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2006/078614 A1 (VENKATESH GOPI M [US]) 13. April 2006 (2006-04-13)<br>* Absatz [0065] *<br>* Beispiel 1 * | 1-5,7-14<br>1-15 | INV.<br>A61K9/20<br>A61K31/135<br>A61K31/4402 |
| E | WO 2017/105852 A1 (PROCTER & GAMBLE [US]) 22. Juni 2017 (2017-06-22)<br>* Seite 26 - Seite 27 *<br>* Ansprüche 1-15 * | 1,6-15 | |
| X<br>Y | CN 102 488 681 A (UNIV SOUTHWEST) 13. Juni 2012 (2012-06-13)<br>* Beispiele 1-5 *<br>* Ansprüche 1-5 * | 1,2,4,5,7,10-13<br>1-15 | |
| X<br>Y | EP 2 500 016 A1 (ESTEVE LABOR DR [ES]) 19. September 2012 (2012-09-19)<br>* Beispiel 2 * | 1,6,8,11-15<br>1-15 | |
| X<br>Y | US 2006/115529 A1 (JEONG SEONGHOON [US] ET AL) 1. Juni 2006 (2006-06-01)<br>* Beispiele 12-15 * | 1,8,11-14<br>1-15 | RECHERCHIERTE SACHGEBIETE (IPC) |
| X<br>Y | US 2011/318411 A1 (LUBER JOSEPH R [US] ET AL) 29. Dezember 2011 (2011-12-29)<br>* Beispiel 3 * | 1,7,11-14<br>1-15 | A61K |
| Y | US 2003/099701 A1 (TAKAISHI YUUKI [JP] ET AL) 29. Mai 2003 (2003-05-29)<br>* Absatz [0043] *<br>* Absatz [0058] - Absatz [0062] *<br>* Tabelle 4 * | 1-15 | |

Der vorliegende Recherchenbericht wurde für alle Patentansprüche erstellt

2

| Recherchenort | Abschlußdatum der Recherche | Prüfer |
|---|---|---|
| München | 5. Oktober 2017 | Sindel, Ulrike |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 17 00 0750

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-10-2017

| Im Recherchenbericht angeführtes Patentdokument | Datum der Veröffentlichung | Mitglied(er) der Patentfamilie | Datum der Veröffentlichung |
|---|---|---|---|
| US 2006078614 A1 | 13-04-2006 | KEINE | |
| WO 2017105852 A1 | 22-06-2017 | US 2017172948 A1 | 22-06-2017 |
| | | WO 2017105852 A1 | 22-06-2017 |
| CN 102488681 A | 13-06-2012 | KEINE | |
| EP 2500016 A1 | 19-09-2012 | AR 085431 A1 | 02-10-2013 |
| | | EP 2500016 A1 | 19-09-2012 |
| | | EP 2536401 A1 | 26-12-2012 |
| | | WO 2012126770 A1 | 27-09-2012 |
| US 2006115529 A1 | 01-06-2006 | US 2006115529 A1 | 01-06-2006 |
| | | WO 2006101536 A1 | 28-09-2006 |
| US 2011318411 A1 | 29-12-2011 | US 2011318411 A1 | 29-12-2011 |
| | | US 2011319441 A1 | 29-12-2011 |
| | | US 2017035656 A1 | 09-02-2017 |
| US 2003099701 A1 | 29-05-2003 | US 2003099701 A1 | 29-05-2003 |
| | | US 2005100599 A1 | 12-05-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- Das Europäische Arzneibuch (Ph.Eur.). 2014, vol. 1, 1206
- Schmelztabletten eine Zerfallszeit gemäß Ph.Eur. 2014, vol. 1, 397ff
- Europäischen Arzneibuch. 2014, vol. 1, 412
- **A. RITSCHEL et al.** Die Tablette. Editio-Cantor-Verlag, 2002
- Die Zerfallszeit ist gemäß Ph.Eur. 2014, vol. 1, 397 ff
- Die Friabilität ist gemäß Ph.Eur. 2014, vol. 1, 411 ff